## Europäisches Patentamt
## European Patent Office
## Office européen des brevets

(19)

(11) Numéro de publication: **0 048 209**
**B1**

(12)

# FASCICULE DE BREVET EUROPEEN

(45) Date de publication du fascicule du brevet: **08.01.86**

(21) Numéro de dépôt: **81401434.6**

(22) Date de dépôt: **15.09.81**

(51) Int. Cl.⁴: **G 01 N 33/92,** G 01 N 33/96, A 61 K 35/16

(54) **Procédé de préparation de solutions de lipoprotéines à partir de résidus de délipidation des plasmas et sérums sanguins, produits nouveaux obtenus, et leurs applications.**

(30) Priorité: **16.09.80 FR 8019935**

(43) Date de publication de la demande: **24.03.82 Bulletin 82/12**

(45) Mention de la délivrance du brevet: **08.01.86 Bulletin 86/02**

(84) Etats contractants désignés: **CH DE FR GB LI NL**

(56) Documents cités:
**EP-A-0 013 814**
**EP-A-0 031 955**
**FR-A-2 229 966**
**US-A-3 382 227**
**US-A-3 859 047**
**US-A-3 998 946**
**US-A-4 045 176**
**US-A-4 216 117**

**Le dossier contient des informations techniques présentées postérieurement au dépôt de la demande et ne figurant pas dans le présent fascicule.**

(73) Titulaire: **BIOMERIEUX, Société Anonyme dite Marcy l'Etoile F-69260 Charbonnières-les-Bains (FR)**

(72) Inventeur: **Trouyez, Gérard Marcy l'Etoile F-69260 Charbonnieres les Bains (FR)**

(74) Mandataire: **Combe, André et al CABINET BEAU DE LOMENIE 55 rue d'Amsterdam F-75008 Paris (FR)**

(56) Documents cités:
**Z. KLIN. CHEM. U. KLIN BIOCHEM., vol. 6, no. 3, 1968, W. STEPHAN et al.: "Adsorption von Lipoproteiden", pages 186-190**

## Description

La présente invention concerne un procédé de traitement des résidus de délipidation des plasmas et sérums sanguins et de stabilisation des solutions de lipoprotéines récupérées à partir de ces résidus; elle concerne également les produits nouveaux obtenus et leurs applications.

L'obtention et l'utilisation des sérums de contrôle ou de calibration à taux élevé de lipides posent des problèmes complexes. En effet, la présence de lipides entraîne une turbidité élevée de ces sérums les rendant impropres à l'usage diagnostique.

Mais la nécessité d'augmenter les taux de lipides et partiuclièrement de cholestérol des sérums de contrôle et de calibration a suscité un certain nombre de techniques:

"solubilisation" du cholestérol à l'aide d'agents tensio-actifs (FOX. C. Diagonostic aid, brevet des Etats-Unis d'Amérique n°3 260 648 du 12 juillet 1966),

utilisation de dérivés solubles du cholestérol (B. KLEIN brevet des Etats-Unis d'Amérique n°3 859 047 du 7 janvier 1975);

utilisation de lipoprotéines bovines séparées par précipitation par le sulfate de dextrane (G. J. PROKSCH et D. P. BONDERMAN Clinical Chemistry 1976, 22/8, 1302-1305);

utilisation de protéines humaines (J. M. WILLIAMS, L. TAYLOR, M. KUCHMACK, R. F. WITTER, Clinica Chimica Acta 1970 (28) 247.253);

utilisation d'échangeurs d'ions tels que polysaccharides réticulés, sulfatés, insolubles pour la séparation les lipoprotéines (Brevets de la Development Finance Corporation of New Zealand, n°180 199 du 4 mars 1976).

Actuellement, de nombreux auteurs soulignent la nécessité d'utiliser, pour la calibration et le contrôle du bilan lipidique des sérums ou étalons dont la sensibilité vis-à-vis de l'attaque enzymatique et les propriétés électrophorétiques soient très proches du sérum humain. Les techniques utilisant un cholestérol "solubilsé" ne sont donc pas utilisables dans cette optique.

La technique consistant à précipiter les lipoprotéines par la sulfate de dextrane sur un autre polymère soluble a comme inconvénient la difficulté d'éliminer ultérieurement la polymère utilisé.

L'utilisation déchangeurs d'ions est difficile à transposer à l'échelle industrielle.

Enfin, vu la dénaturation les lipoprotéines lors de la lyophilisation, il importe de trouver un procédé de stabilisation des lipoprotéines.

La technique de délipidation des sérums par l'acide silicique décrite par W. STEPMAN et L. ROKA (Z. Klin. chem. u. Klin. Biochem., 1968 6 (3) 186-190) était utilisée pour préparer des sérums dont la composition (hormis les lipoprotéines) est stable et proche de celle d'un sérum non traité. Toutefois, les résidus lipidiques de cette délipidation n'étaient pas utilisés.

Aussi, la présente invention a pour but, d'une part, de récupérer ces sous-produits pour les utiliser dans le domaine diagnostique pharmaceutique ou cosmétique, d'autre part, de stabiliser les solutions de lipoprotéines récupérées afin d'obtenir des solutions de calibration ou de contrôle n'ayant par les inconvénients des sérums lyophilisés.

L'invention concerne un procédé de préparation de solutions stables de lipoprotéines à partir des produits bruts de délipidation du plasma ou du sérum sanguin par l'acide silicique ou ses dérivés et l'utilisation des solutions ainsi obtenues dans le domaine diagnostique, pharmaceutique ou cosmétique.

Le procédé de l'invention est caractérisé en ce qu'on opère en deux étapes:

la première consistant à traiter les produits bruts de délipidation de façon à obtenir une solution saline de lipoprotéines;

la seconde consistant à stabiliser la solution ainsi obtenue.

La matière première utilisée est le produit brut de délipidation des sérums et plasmas bovins ou humains, selon le procédé connu d'adsorption sur l'acide silicique.

Dans ce procédé le sérum ou le plasma est mis en contact avec de la silice colloïdale (par exemple Aérosil® 380 vendu par DEGUSSA R.F.A.) à une concentration de 0,2 à 20 %, de préférence de 1 à 5 %. On agite après avoir ajusté le pH à des valeurs physiologiques (entre pH 7 et 7,5).

La première étape du procédé selon l'invention a pour objet d'assurer la séparation, dans le précipité obtenu par précipitation du plasma ou du séum sanguin avec l'acide silicique, de l'acide silicique d'avec les lipoprotéines et de solubiliser ces derniers.

Cette première étape est mise en oeuvre en:

a) mélangeant en quantités (ne poids) sensiblement égales ledit précipité avec de l'eau, à ajouter au mélange une quantité de chlorure de sodium sensiblement égale à la moitié (en poids) de l'eau ajoutée et à ajuster le pH du mélange à une valeur déterminée,

b) récupérant le liquide obtenu pour, par dialyse par exemple, obtenir une solution contenant environ 1 à 30 g/l de chlorure de sodium et 1 à 100 g/l de lipoprotéines.

On notera:

— que dans le mélange initial entre le précipité et l'eau, les quantités relatives de ces deux éléments ne sont pas indifférentes. Un excès d'eau risque d'entrainer une solubilisation partielle de faibles quantités d'acide silicique que, dans certaines applications des produits de l'invention, peuvent se révéler indésirables. Une quantité d'eau insuffisante conclut à un mélange présentant une viscosité trop élevée. Il a été déterminé expérimentalement que la quantité d'eau à utiliser est sensiblement égale, en poids, à la quantité de précipité traité,

— que l'on utilise dans le stade a) une quantité très importante de chlorure de sodium en utilisant le phénomène selon lequel la solubilite du chlorure de sodium dans l'eau est fortement augentée du fait de la présence du précipité. Si la proportion de chlorure de sodium est insuffisante on n'arrive pas à séparer complètement les matières organiques (lipoprotéines) d'avec l'acide silicique. Il a été déterminé que la quantité de chlorure de sodium à utiliser était d'environ la moitié (en poids) de la quantité d'eau présente.

— que dans le stade a) de l'invention le pH des solutions doit être déterminé avec une grande précision; cependant ce pH dépend de l'origine des plasmas ou sérums utilisés. Dans le cas de sérum ou plasma d'origine bovine on ajustera le pH à la valeur de 10,5±0,1; dans le cas de sérum ou plasma d'origine humaine on ajustera le pH à la valeur de 9,75±0,2.

Les solutions salines de lipoprotéines obtenues après cette première étape du procédé sont stables à condition d'être conservées à l'abri de la lumière, à l'état aseptique et à une basse température (par exemple 4°C). Elles peuvent donc être utilisées directement notamment dans l'industrie pharmaceutique (galénique) ou cosmétique. Cependant en veu de certains usages et notamment en vue de la préparation de solutions étalons, ces solutions sont avantageusement stabilisées dans la seconde étape du procédé selon l'invention.

Cette seconde étape consiste à ajouter aux solutions salines de lipoprotéines (obtenues dans la première étape) un antiseptique, le bromonitrodioxan, et éventuellement un tampon.

L'antiseptique utilisé pour stabiliser les solutions de lipoprotéines selon l'invention est le bromonitrodioxan connu pour ses propriétés antiseptiques, qui ne dénature pas les lipoprotéines, et qui possède également des propriétés anti-oxydantes en antibactériennes.

Le bromo-nitrodioxane ou "Bronidox" (marque déposée par la Société HENKEL) est avantageusement utilisé à une concentration de 0,1 à 1%,

Il est parfois souhaitable de tamponner les solutions obtenues (qui sont à un pH compris entre 6,5 et 8,5) pour obtenir des solutions à pH stable déterminé de préférence 7.

Diverses catégories de tampons peuvent être utilisées:

— des tampons tels que le tampon phosphate; la molarité peut alors varier de 10 à 500 mmoles/ml, le pH étant compris entre 6,5 et 8,5 et de préférence egal à 7;
— des tampons mixtes tels que phosphate-carbonate dans les mêmes conditions que le tampon phosphate;
— des tampons aminés substitués ou non;
— des tampons "zwitterioniques", de tels tampons étant d'excellents chélateurs de cations métalliques qui entraînent la précipitation des lipoprotéines. Ainsi, il est possible d'utiliser des tampons dont la fonction basique est une amine et la fonction acide, une fonction acide carboxylique ou acide sulfonique, tels que:

. le MPS ou acide morpholino-3-propane sulfonique;
. le PIPES ou acide 1,4-pipérazine diéthanesulfonique;
. le TES acide N-tris (hydroxyméthyl)-méthyl-2-amino-2-éthane sulfonique; ou tout autre tampon zwitterionique de pH adapté.

La nature des lipoprotéines ainsi récupérées varie selon la nature du sérum ou du plasma utilisé comme matière première: les sérums ou plasmas bovins sont une excellente source de lipoprotéines de haute densité; les sérums ou plasmas humains permettent d'obtenir des solutions contenant diverses classes de lipoprotéines: de très basse, intermédiaire, basse et haute densités.

Les utilisations des solutions de lipoprotéines obtenues selon l'invention sont les suivantes:

les solutions salines de lipoprotéines peuvent servir à surcharger des sérums de calibration ou de contrôle lyophilisés. Ces sérums de contrôle ou de calibration peuvent servir pour l'étalonnage ou le contrôle des dosages des constituants suivants dans le sang: triglycérides, cholestérol, phospholipides, apoprotéines ainsi que pour le dosage des mêmes constituants dans les diverses fractions de lipoprotéines, ces dosages pouvant être effectués par des procédés physiques, chimiques, immunologiques ou radioimmunologiques;

les solutions stabilisées de lipoprotéines peuvent, outre les usages définis ci-dessu, être utilisées comme sérum de contrôle pour l'électrophorèse, l'ummunodiffusion, l'ummunoprécipitation, l'électroimmunodiffusion ou tout autre procédé enzymatique immunologique;

les solutions stabilisées peuvent être utilisées comme substrat pour les dosages des activités enzymatiques impliquées dans le métabolisme lipidique, telles que:
lipoprotéine-lipase;
lécithine, cholestérol-acyltransférase, lipase;
ou tout autre activité enzymatique nécessitant l'un quelconque des constituants énoncés ci-dessus;

de plus, les solutions salines de lipoprotéines ou les solutions stabilisées de lipoprotéines peuvent être utilisées comme source de lipides ou de lipoprotéines dans l'industrie cosmétique;

enfin, les solutions de lipoprotéines, stabilisées ou non, peuvent être utilisées pour l'élaboration de crèmes, pommandes, lotions ou tout autre produit à usage cosmétique ou pharmaceutique.

**Revendications**

1. Procédé de préparation de solutions stables de lipoprotéines à partir des précipités obtenus par délipidation du plasma ou du sérum sanguin par de l'acide silicique ou ses dérivés et comprenant les étapes suivantes:

— de mélange dudit précipité avec un poids sensiblement égal d'eau et une quantité de chlorure de sodium sensiblement égale à la moitié, en poids, de l'eau utilisée et ajustement du pH du mélange à une valeur déterminée,

— de récupération du liquide obtenu dans l'opération précédente et traitement de celui-ci, par exemple, as dialyse en vue d'obtenir une solution aqueuse contenant 1 à 30 g/l environ de chlorure de sodium et 1 à 100 g/l environ de lipoprotéine, et caractérisé en ce que l'on procède à la stabilisation des solutions salines obtenues par addition à ces solutions de bromonitrodioxanne et éventuellement d'un· tampon.

2. Procédé selon la revendication 1, caractérisé en ce que l'on utilise comme produit de départ un précipité obtenu par traitement d'un plasma ou sérum sanguin de bovin et que le pH du mélange est ajusté à 10,5±0,1.

3. Procédé selon la revendication 1, caractérisé en ce que l'on utilise comme produit de départ un précipité obtenu par traitement d'un plasma ou sérum humain et que le pH du mélange est ajusté à 9,75±0,2.

4. Solutions salines stables de lipoprotéines obtenues par le procédé de la revendication 1, caractérisées en ce qu'elles contiennent de l'eau, d'environ 1 à environ 30 g/l de chlorure de sodium et d'environ 1 à environ 100 g/l de lipoprotéine provenant d'une mise en solution des précipités obtenus par délipidation des plasmas ou sérums sanguins par de l'acide silicique ou ses dérivés et du bromonitrodioxanne.

5. Solutions salines stables selon la revendication 4, caractérisées en ce qu'elles contiennent un tampon.

6. Application des solutions salines stables selon les revendications 4 ou 5, caractérisée en ce que ces solutions servent à surcharger des sérums de calibration ou de contrôle, ces sérums étant utilisés notamment pour l'étalonnage ou le contrôle de triglycérides, cholestérol, phospholipides, apoprotéines ou des dosages des mêmes constituant dans les diverses fractions de lipoprotéines, les dosages pouvant être effectués par des procédé physiques, chimiques, enzymatiques, immunologiques, radioimmunologiques ou la combinaison de ces procédés.

7. Utilisation des solutions liquides stabilisées de lipoprotéines selon les revendications 4 ou 5 comme sérum de calibration ou de contrôle liquide pour le dosage des triglycérides, phospholipides, cholestérol, apoprotéines, lipoprotéines, les dosages pouvant être effectués selon les techniques décrites dans la revendication 6, caractérisée en ce que la composition de ces solutions est stable dans le temps, similaire à celle d'un sérum humain, et peut être ajustée dans les domaines des valeurs normales ou pathologiques.

8. Utilisation des solutions liquides de lipoprotéines selon les revendications 4 ou 5, comme substrat pour les dosages des activités enzymatiques impliquées dans le métabolisme lipidique ou tout autre activité enzymatique nécessitant l'un quelconque des constituants énoncés dans la revendication 6.

9. Utilisation des solutions salines de lipoprotéines ou des solutions stabilisées de lipoprotéines selon les revendications 4 ou 5 comme sources de lipides ou de lipoprotéines, dans l'industrie cosmétique ou pharmaceutique.

**Patentansprüche**

1. Verfahren zur Herstellung von stabilen Lipoproteinlösungen aus durch Entfettung von Blutplasma oder -serum mit Kieselsäure oder ihren Derivaten in den folgenden Stufen erhaltenen Rückständen:

— Vermischung des Niederschlags mit einer im wesentlichen gleichen Gewichtsmenge Wasser und einer praktisch der Hälfte der verwendeten Gewichtsmenge an Wasser entsprechenden Natriumchloridmenge une Einstellung des pH-Wertes des Gemisches auf einen vorbestimmten Wert,

— Gewinnung und Behandlung der in der vorhergehenden Stufe erhaltenen Flüssigkeit, beispielsweise durch Dialyse zur Herstellung einer wäßrigen Lösung mit einem Gehalt an etwa 1 bis 30 g/l Natriumchlorid und etwa 1 bis 100 g/l Lipoprotein, gekennzeichnet durch die Stabilisierung der erhaltenen Salzlösungen durch Zugabe von Bromnitrodioxan und ggf eines tampons.

2. Verfahren nach Anspruch 1, gekennzeichnet durch die Verwendung eines durch Behandlung von Rinderblutplasma oder -serum erhaltenen Rückstands als Ausgangsprodukt und die Einstellung des pH-Wertes des Gemisches auf 10,5 ±0,1.

3. Verfahren nach Anspruch 1, gekennzeichnet durch die Verwendung eines durch Behandlung von Humanblutplasm oder -serum erhaltenen Rückstands und Einstellung des pH-Wertes des Gemisches auf 9,75±0,2.

4. Stabile Lipoprotein-Salzlösungen, erhältlich gemäß Anspruch 1, dadurch gekennzeichnet, daß sie Wasser, etwa 1 bis 30 g/l Natriumchlorid und etwa 1 bis 100 g/l Lipoportein enthalten, das durch Lösung der durch Entfettung von Blutplasma oder -serum mit Kieselsäure oder ihrer Derivaten und Bromnitrodioxan erhaltenen Rückstande erhalten wurde.

5. Stabile Salzlösungen nach Anspruch 4, dadurch gekennzeichnet, daß sie ein Tampon enthalten.

6. Verwendung der stabilien Salzlösungen nach Anspruch 4 oder 5 zur Überladung von Eichsera oder Kontrollsera, wobei diese Sera insbesondere für die Eichung oder die Kontrolle von Triglyceriden, Cholesterin, Phospholipiden, Apoproteinen oder zur Bestimmung dieser Bestandteile in verschiebdenen Lipoproteinfraktionen verwendet werden und die Bestimmungen physikalisch, chemisch, enzymatisch, immunologisch und/oder radioimmunologisch durchgeführt werden könne.

7. Verwendung der stabilen Lipoproteinlösungen nach Anspruch 4 oder 5 als Eichserum oder Kontrollserum zur Bestimmung von Triglyceriden, Phospholipiden Cholesterin, Aproteinen, Lipoproteinen, wobei die Bestimmungen gemäß Anspruch 6 durchgeführt werden könne, dadurch gekennzeichnet, daß die Zusammensetzung der Lösungen, wie die von Humanserum, zeitlich stabil ist und auf Normalwerte oder pathologische Werte eingestellt werden kann.

8. Verwendung der Lipoproteinlösungen nach Anspruch 4 oder 5 als Substrat zur Bestimmung der enzymatischen Aktivitäten des Lipidmetabolismus oder von anderen enzymatischen Aktivitäten, die einen der in Anspruch 6 aufgeführten Bestandteile benötigen.

9. Verwendung der Lipprotein-Salzlösungen oder stabilen Lipoproteinlösungen gemäß Anspruch 4 oder 5 als Lipododer Lipoproteiquelle in der Kosmetik- oder pharmazeutischen Industrie.

**Claims**

1. Process for preparing stable solutions of lipoproteins from precipitates obtained by removing the lipids of plasma or blood serum by silicic acid or its derivatives, and comprising the following steps,

mixing the precipitate with a substantially equal weight of water and a quantity of sodium chloride substantially equal to half, by weight, of the water used and adjusting the pH of the mixture to a determined value,

recovering the liquid obtained in the preceding steps and treating it, for example by dialysis, with the view to obtaining an aqueous solution containing about 1 to 30 g/litre of sodium chloride, and about 1 to 100 g/litre of lipoproteins, and characterized in that:

a stabilization of the resulting saline solutions is carried out by adding to said solutions bromonitrodioxane and optionally a buffer.

2. Process according to claim 1, characterized in that a precipitate obtained by treating a plasma or blood serum of a bovine, is used as starting product, and in that the pH is adjusted to 10.5±0.1.

3. Process according to claim 1, characterized in that a precipitate obtained by treating a plasma or human serum is used as starting product, and in that the pH of the mixture is adjusted to 9.75±0.2.

4. Stable saline solutions of lipoproteins obtained by the process according to claim 1, characterized in that they contain, about 1 to about 30 g/litre of sodium chloride and about 1 to about 100 g/litre of lipoproteins resulting from a dissolution of the precipitates obtained by removal of lipids of blood plasma or serums by silicic acid or its derivatives, and bromonitrodioxane.

5. Stable saline solutions according to claim 4, characterized in that they contain a buffer.

6. Application of stable saline solutions according to claims 4 or 5, characterized in that said solutions are useful for overcharging calibration or control serums, said serums being used particularly for standardizing or controlling triglycerides, cholesterol, phospholipides, apoproteins, or assays of the same constituents in the different fractions of lipoproteins, the assays can be carried out by physical, chemical, enzymatic, immunological, radioimmunological processes, or the combination of these processes.

7. Use of stabilized liquid solutions of lipoproteins according to claims 4 or 5, as liquid calibration or control serum for the assay of triglycerides, phospholipides, cholesterol, apoproteins, lipoproteins, the assays can be carried out according to the techniques described in claim 6, characterized in that the composition of these solutions is stable in time, is identical to that of a human serum, and can be adjusted in the ranges of normal or pathological values.

8. Use of liquid solutions of lipoproteins according to claims 4 or 5, as substrate for assaying the enzymatic activities involved in the lipid metabolism or any other activity requiring any one of the constituents mentioned in claim 6.

9. Use of saline solutions of lipoproteins or stabilized solutions of lipoproteins according to claims 4 or 5, as sources of lipid or lipoproteins, in the cosmetic or pharmaceutical industry.